# EUROPEAN PATENT SPECIFICATION

(11) **EP 1 803 419 B1**
(45) Date of publication and mention of the grant of the patent: **05.03.2014**
(21) Application number: 07250009.3
(22) Date of filing: 03.01.2007
(51) Int. Cl.: A61F 2/07

(54) **Prosthetic stent graft for treatment of aortic aneurysm**
Stentgraftprothese zur Behandlung eines Aortenaneurysmas
Stent-greffe prothétique pour le traitement des anévrismes de l'aorte

(30) Priority: 03.01.2006 US 324682
(43) Date of publication of application: 04.07.2007
(73) Proprietor: Cordis Corporation, Miami Lakes, FL 33014 (US)
(72) Inventor: Majercak, David C., Stewartsville NJ 08886 (US); Park, Jin S., Parsippany NJ 07054 (US)
(74) Representative: Belcher, Simon James

(56) References cited:
- EP-A2- 1 029 518
- WO-A-97/12562
- WO-A1-95/21592

## Description

The invention relates to the field of medical devices, and more specifically to a prosthesis for the treatment of vascular disease, particularly abdominal aortic aneurysm.

Vascular disease is a leading cause of premature mortality in developed nations, often presenting as a vascular aneurysm. A vascular aneurysm is a localized dilation of a vessel wall, due to thinning or weakness of the wall structure, or separation between layers of the vessel wall. If untreated, the aneurysm may burst and haemorrhage uncontrollably. Aneurysms are particularly dangerous and prevalent in the aorta, because the aorta supplies blood to all other areas of the body, and because the aorta is subject to particularly high pressures and stresses accordingly. Rupture of an aortic aneurysm is the 15^{th} leading cause of death the United States, afflicting 5% of older men.

Aortic aneurysms are described by their position. They are either thoracic, generally between the aortic arch and the junction of the left and right renal arteries, or abdominal, between the junction of the renal arteries and the branch of the iliac arteries.

It is known to treat aortic aneurysms surgically where blood pressure control medication is unsuccessful at arresting growth of the aneurysm. Surgery often involves the insertion of a vascular stent graft to exclude the aneurysm and carry blood past the dilated portion of the vessel, relieving the pressure on the aneurysm. Designing a viable stent graft for the treatment of abdominal aortic aneurysm (AAA) is particularly challenging, in part because the graft must branch to follow the shape of the abdominal aorta to carry blood into the separate iliac arteries without obstruction.

Moreover, it would be advantageous to design a stent graft that is collapsible to facilitate percutaneous insertion by minimally invasive surgical techniques. Toward this end, certain stent grafts for the treatment of AAA are delivered in parts. One part of the stent graft forms a generally inverted Y shape, for insertion into the trunk of the abdominal aorta, and branching into the iliac arteries. A second part of the stent graft is deployed in the iliac artery and interfaces with the first part.

However, the stent graft must remain in the proper position after deployment and for years of continuous use. Accordingly it would be advantageous to design a multi-part stent graft that can be secured in position by minimally invasive surgical techniques. Further, it would be advantageous for a stent graft to be sizable in situ without compromising positional stability once deployed.

WO 95/21592 describes an aortic graft having trunk and leg portions, wherein the trunk has outlets into which leg portions are inserted. The trunk is generally elastically compliant with an inelastic edge. The leg portions comprise a stent which when inserted and expanded in the trunk outlets are retained by the inelastic portion.

In one aspect, the present invention provides a prosthetic stent graft, as defined in appended claim 1.

The first taper angle of the flow channels is preferably greater than or more preferably equal to the taper angle of the leg section. Preferably, the deployed diameter of the leg section inlet is at least as large or larger than the deployed diameter of the flow channels adjacent the bifurcation point of trunk section. Preferably, the deployed diameter of flow channel outlets are at least smaller than the deployed diameter of the leg section at an equal distance from the leg section inlet as the outlet is from bifurcation point.

The device of the invention can be used in a method of in situ stent graft sizing for the treatment of abdominal aortic aneurysm. As part of the method, a bifurcated trunk section of a stent graft is deployed in the abdominal aorta of a patient. Further, a leg section of the stent graft is deployed with the inlet of the leg section within one of the bifurcated flow channels of the trunk section, the outlet of the leg section extending into the iliac artery. The inlet of the leg section has a deployed diameter at least as large or larger than the outlet of the bifurcated flow channel it is deployed within.

Embodiments of the invention will now be described by way of example with reference to the accompanying drawings, in which:
Fig. 1 shows a bifurcated trunk section of a stent graft according to the invention;
Fig. 2 illustrates a leg section of the stent graft according to the invention;
Fig. 3 illustrates a bifurcated trunk section and leg section of the stent graft shown in Figs. 1 and 2, superimposed over one another at their respected maximum deployed diameters, and at a preferred maximum axial overlap between the sections; and
Fig. 4 illustrates a bifurcated trunk section and leg section of the stent graft shown in Figs. 1 and 2, superimposed over one another at their respected maximum deployed diameters, and at a preferred minimum axial overlap between the sections.

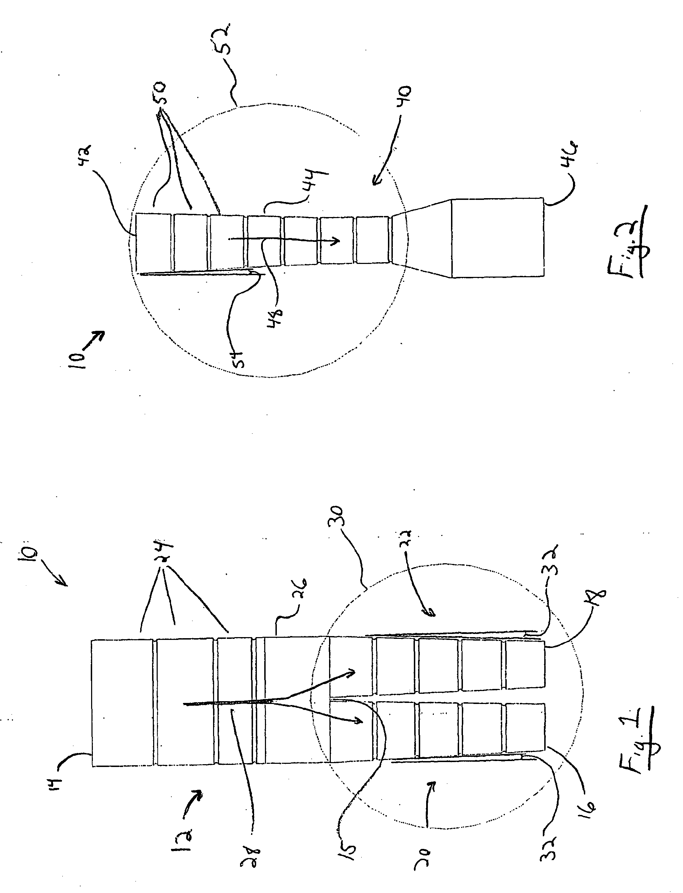

Referring to the drawings, Figs. 1 and 2 show two components of a stent graft 10. The trunk section 12 has an approximately circular inlet 14 at one end, and two approximately circular outlets 16, 18, at an opposite end. Inlet and outlet are derived from the intended direction of blood flow, indicated by arrow 28, through the trunk section 14 when positioned in the abdominal aorta and iliac arteries of a patient. The trunk section 12 bifurcates at point 15 between inlet 14 and outlets 16, 18 into two flow channels, 20, 22, respectively. Preferably, the trunk section 12 is comprised of plural stent segments 24 along its length, secured to and joined by a vascular graft 26 which surrounds the segments 24 and defines the flow through the trunk section 12. However, a unitary stent structure may be used as well, in addition to more or fewer segments 24 of varying length than shown in this exemplary embodiment. In a preferred embodiment, stent segments are approximately 1 cm in axial length.

Referring now to the bifurcated section highlighted by circle 30, according to the exemplary embodiment, the flow channels 20, 22 are each tapered, and reduce in diameter along their length between point 15 and outlets 16, 18.

Turning now to Fig. 2, stent graft 10 includes a leg section, generally 40. Leg section 40 has a generally circular inlet 42. Graft section 44 defines a flow path between inlet 42 and a generally circular outlet 46. Inlet and outlet are derived from the intended direction of blood flow, indicated by arrow 48, through the leg section 40 when positioned in the abdominal aorta and iliac arteries of a patient. Preferably, the leg section 40 is comprised of plural stent segments 50 along its length, secured to and joined by vascular graft 44 which surrounds the segments 50 and defines the flow through the leg section 40. However, a unitary stent structure may be used as well, in addition to more or fewer segments 50 of varying length than shown in this exemplary embodiment.

Referring now to the inlet section highlighted by circle 52, according to the exemplary embodiment, the leg section 40 is tapered, and reduces in diameter along its length at least between inlet 42 and some arbitrary point along its length. As shown in the exemplary embodiment, the leg portion 40 also expands in diameter from a minimum diameter along its length to a larger diameter at the generally circular outlet 46. However, this is merely optional. This expanded deployed diameter may be constrained by the vessel in which the outlet 46 of the leg portion 40 is deployed. Alternately, the taper may cease along its length, at which point the diameter may be arbitrarily set to suit the needs of the particular application, including continuing at a uniform diameter to the outlet 46. Alternatively, the minimum diameter may itself occur at outlet 46.

Moreover, the taper angle 32, 54 is provided to improve fluid flow through the graft 10, by reducing flow turbulence. Reduced turbulence reduces the fluid pressures in the graft 10, thereby reducing stress on the graft 10, and particularly stent segments 24, 50 thereof. Accordingly the graft 10 is more resilient and enjoys longer service life.

Preferably, the stent segments 24, 50 forming the structure of stent graft 10 comprise a shape-memory material, more preferably Nitinol. Having been shape-set to a deployed diameter, the stent graft may be crimped to a smaller delivery diameter for percutaneous delivery by minimally invasive surgical techniques.

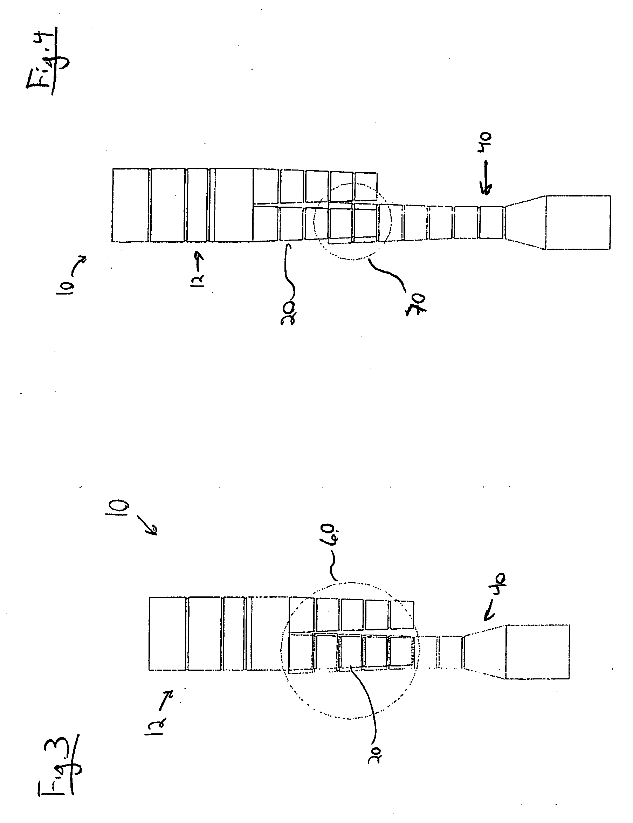

In use, the trunk section 12 of stent graft 10 would first be deployed in the abdominal aorta of the patient. Exposed to the heat of the patient's body temperature, and/or released from the constraint of a delivery apparatus, the stent graft 10 would expand to its deployed size, and fix against the walls of the aorta. Subsequently, one or two leg sections 40 would be deployed inside either or both of flow channels 20, 22. Preferably, the inlet 42 of leg section 40 would be deployed as close as practicable to the bifurcation point 15 of the trunk section 12. This affords the maximum overlap between the trunk section 12 and the leg section 40, which increases the pull-out force necessary to dislodge the leg section. Accordingly, the leg section 40 is more resistant to dislodgment. However, in practice, *in situ* sizing of the length of the stent graft 10 is often necessary, and the length of overlap between trunk section 12 and leg section 40 can be reduced as necessary, as shown in Figs. 3 and 4.

The deployed diameter of the inlet 42 of leg section 40 is at least as large or larger than the deployed diameter of flow channels 20, 22 at their largest dimensions adjacent the bifurcation point 15 of trunk section 12. Additionally, the taper angle 32 of flow channels 20, 22, is at least approximately equal to or greater than the taper angle 54 of the tapered portion of leg section 40. Preferably, taper angle 32 and taper angle 54 are approximately equal. Therefore, presuming maximum overlap of the leg section 40 and flow channel 20, 22, the deployed diameter of the outlets 16, 18, is as least the same size or smaller than the corresponding deployed diameter of leg section 40 an equal distance from inlet 42 as outlet 16, 18 is from bifurcation point 15.

Accordingly, when a leg section 40 is inserted into a flow channel 20, 22, the deployed diameter of the leg section 40 would seek to exceed the inner deployed diameter of the flow channel 20, 22, but for containment of the leg section 40 by the flow channel 20, 22. The pull-out force necessary to dislodge the leg section from the flow channel increases with the amount by which the deployed diameter of the leg portion 40 exceeds the deployed diameter of the flow channel 20, 22, and also with the length of overlap between the leg section 40 and the flow channel 20, 22. Further, the pull-out force necessary to dislodge the leg section 40 increases with the taper angle 32.

Referring now to Figs. 3 & 4, stent graft 10 is illustrated with leg section 40 superimposed over flow channel 20, both in fully deployed diameters, at a preferred maximum (Fig. 3) and preferred minimum (Fig. 4) amounts of axial overlap length between the two. It will be appreciated that more or overlap may be used, within the dimensional constraints of the leg section 40 and flow channel 20. In Fig. 3, it will be seen that the diameter differential between the leg section 40 and the flow channel 20 at any given point along their lengths is reduced by increasing amount of overlap, in conjunction with the taper angles 32, 54, highlighted by circle 60. Comparing now to Fig. 4, the axial overlap between leg section 40 and flow channel 20 is highlighted by circle 70. Although the lengths of the overlap is reduced as compared to Fig. 3, the differential in deployed diameter between leg section 40 and flow channel 20 is increased, in part due to the taper angles 32, 54. Any decrease in pull out force in the configuration of Fig. 4 because of the reduced overlap length is compensated for by the increase in pull out force by the increased differential in deployed diameter. Therefore, a surgeon can size the overall length of the stent graft 10 *in situ* without compromising pull-out force of the leg section 40.

## Claims

1. A prosthetic stent graft (10) comprising:
a trunk section (12) having one or more stent segments along its entire length and a bifurcated vascular graft, the trunk section further having a first inlet (14) and first (20) and second (22) flow channels in fluid communication with first (16) and second (18) outlets, the bifurcated vascular graft supported by one or more of the stent sections (24) along its length;
each of the first (20) and second (22) flow channels having a first taper angle (32), whereby the respective flow channel decreases in cross-sectional area along its length between a bifurcation point (15) in the trunk section (12) to an outlet (16,18) of the flow channel (20, 22);
a leg section (40) having a tubular vascular graft having a second inlet (42) with a deployed diameter at least as large or larger than a deployed diameter of the first (16) or second (18) outlet of the first (20) or second (22) flow channel, respectively, and a third flow channel (44) in fluid communication with a third outlet (46), the tubular vascular graft supported by second one or more stent sections (50) along its length; and
the leg section (40) having a second taper angle (54), whereby the third flow channel (44) decreases in cross-sectional area along its length between the second inlet (42) and a location along the length of the leg section, **characterised in that**
the first (32) and second (54) taper angles are such that the overlap between the leg section (40) and the respective flow channel (20, 22) can be altered so that the length of the prosthetic stent graft (10) can be altered in situ without compromising the pull-out force of the leg section (40).

2. The prosthetic stent graft according to claim 1, in which the first one or more stent sections (24) or the second one or more stent sections (50) comprise a shape memory material.

3. The prosthetic stent graft according to claim 2, in which the shape memory material comprises Nitinol.

4. The prosthetic stent graft according to claim 1, in which the first taper angle (32) is greater than or equal to the second taper angle (54).

5. The prosthetic stent graft according to claim 1, in which the first taper angle (32) is approximately equal to the second taper angle (54).

6. The prosthetic stent graft according to claim 1, in which the deployed diameter of the second inlet (42) of the leg section (40) is at least as large or larger than the deployed diameter of the first (20) or second (22) flow channel adjacent the bifurcation point (15) of trunk section (12).

7. The prosthetic stent graft according to claim 1, in which a deployed diameter of first (16) or second (18) outlet is as least the same as or smaller than the deployed diameter of the leg section at an equal distance from the second (42) inlet as the first (16) or second (18) outlet is from bifurcation point (15),

8. The prosthetic stent graft according to claim 1, in which the bifurcated vascular graft of the trunk section (12) surrounds and is secured to the first one or more stent sections (24).

9. The prosthetic stent graft according to claim 1, in which the tubular vascular graft of the leg section (40) surrounds and is secured to the second one or more stent sections (50).

## Patentansprüche

1. Stentgraftprothese (10), die Folgendes aufweist:
einen Stammabschnitt (12) mit einem oder mehreren Segmenten entlang dessen gesamter Länge und einen gabelförmigen Gefäßgraft, wobei der Stammabschnitt ferner einen ersten Einlass (14) und einen ersten (20) und zweiten (22) Strömungskanal in Fluidverbindung mit einem ersten (16) und zweiten (18) Auslass aufweist, und der gabelförmige Gefäßgraft von einem oder mehreren der Stentabschnitte (24) entlang dessen Länge gestützt wird;
wobei jeder der ersten (20) und zweiten (22) Strömungskanäle einen ersten Konuswinkel (32) aufweist, wobei der jeweilige Strömungskanal in einer Querschnittsfläche entlang dessen Länge zwischen einem Gabelpunkt (15) in dem Stammabschnitt (12) zu dem Auslass (16, 18) des Strömungskanals (20, 22) abnimmt;
einen Beinabschnitt (40) mit einem röhrenförmigen Gefäßgraft mit einem zweiten Einlass (42), wobei ein entfalteter Durchmesser wenigstens so groß oder größer ist als ein entfalteter Durchmesser des ersten (16) oder zweiten (18) Auslasses des ersten (20) bzw. zweiten (22) Strömungskanals, und einen dritten Strömungskanal (44) in Fluidverbindung mit einem dritten Auslass (46), wobei der röhrenförmige Gefäßgraft entlang dessen Länge von einem oder mehreren zweiten Stentabschnitten (50) gestützt; und
wobei der Beinabschnitt (40) einen zweiten Konuswinkel (54) aufweist, und der dritte Strömungskanal (44) in einer Querschnittsfläche entlang dessen Länge zwischen dem zweiten Einlass (42) und einem Ort entlang der Länge des Beinabschnitts abnimmt,
**dadurch gekennzeichnet, dass**
der erste (32) und der zweite (54) Konuswinkel so ausgebildet sind, dass der Überlapp zwischen dem Beinabschnitt (40) und dem jeweiligen Strömungskanal (20, 22) verändert werden kann, so dass die Länge der Stentgraftprothese (10) in situ verändert werden kann, ohne die Auszugskraft des Beinabschnitts (40) zu beeinträchtigen.

2. Stentgraftprothese nach Anspruch 1, in welcher der eine oder die mehreren ersten Stentabschnitte (24) oder der eine oder die mehreren zweiten Stentabschnitte (50) ein Formgedächtnismaterial aufweisen.

3. Stentgraftprothese nach Anspruch 2, in welcher das Formgedächtnismaterial Nitriol umfasst.

4. Stentgraftprothese nach Anspruch 1, in welcher der erste Konuswinkel (32) größer oder gleich ist wie der zweite Konuswinkel (54).

5. Stentgraftprothese nach Anspruch 1, in welcher der erste Konuswinkel (32) etwa gleich zum zweiten Konuswinkel (54) ist.

6. Stentgraftprothese nach Anspruch 1, in welcher der Entfaltungsdurchmesser des zweiten Einsatzes (42) des Beinabschnitts (40) wenigstens so groß oder größer ist als der entfaltete Durchmesser des ersten (20) oder zweiten (22) Strömungskanals benachbart zu dem Gabelpunkt (15) des Stammabschnitts (12).

7. Stentgraftprothese nach Anspruch 1, in welcher der enfaltete Durchmesser des ersten (16) oder zweiten (18) Auslasses wenigstens gleich oder kleiner ist wie der entfaltete Durchmesser des Beinabschnitts in einem gleichen Abstand zu dem zweiten (42) Einlass wie der erste (16) oder der zweite (18) Auslass von dem Gabelungspunkt (15).

8. Stentgraftprothese nach Anspruch 1, in welcher der gabelförmige Gefäßgraft des Stammabschnitts (12) den einen oder die mehreren ersten Stentabschnitten (24) umgibt und daran befestigt ist.

9. Stentgraftprothese nach Anspruch 1, in welcher der röhrenförmige Gefäßgraft des Beinabschnitts (40) den einen oder die mehreren zweiten Stentabschnitte (50) umgibt und daran befestigt ist.

## Revendications

1. Stent greffe prothétique (10), comprenant :
une section de tronc (12) ayant un ou plusieurs segments de stent sur toute sa longueur et une greffe vasculaire bifurquée, la section de tronc ayant en outre une première admission (14) et des premier (20) et second (22) canaux d'écoulement en communication fluidique avec des première (16) et seconde (18) évacuations, la greffe vasculaire bifurquée étant supportée par une ou plusieurs des sections de stent (24) sur sa longueur ;
chacun des premier (20) et second (22) canaux d'écoulement ayant un premier angle conique (32), moyennant quoi le canal d'écoulement respectif diminue en zone de section transversale sur sa longueur entre un point de bifurcation (15) dans la section de tronc (12) à une évacuation (16, 18) du canal d'écoulement (20, 22) ;
une section de branche (40) ayant une greffe vasculaire tubulaire ayant une seconde admission (42) avec un diamètre déployé au moins aussi grand ou plus grand qu'un diamètre déployé de la première (16) ou seconde (18) évacuation du premier (20) ou second (22) canal d'écoulement, respectivement, et un troisième canal d'écoulement (44) en communication fluidique avec une troisième évacuation (46), la greffe vasculaire tubulaire étant supportée par une ou plusieurs secondes sections de stent (50) sur sa longueur ; et
la section de branche (40) ayant un deuxième angle conique (54), moyennant quoi le troisième canal d'écoulement (44) diminue en zone en section transversale sur sa longueur entre la deuxième admission (42) et un emplacement le long de la longueur de la section de branche,
**caractérisé en ce que** les premier (32) et deuxième (54) angles coniques sont tels qu'ils se chevauchent entre la section de branche (40), et le canal d'écoulement respectif (20, 22) peut être modifié de sorte que la longueur de la stent greffe prothétique (10) peut être modifiée in situ sans compromettre la force de rétraction de la section de branche (40).

2. Stent greffe prothétique selon la revendication 1, dans lequel les premières une ou plusieurs sections de stent (24) ou les deuxièmes une ou plusieurs sections de stent (50) comprennent un matériau de mémoire de forme.

3. Stent greffe prothétique selon la revendication 2, dans lequel le matériau de mémoire de forme comprend le Nitinol.

4. Stent greffe prothétique selon la revendication 1, dans lequel le premier angle conique (32) est plus grand ou égal au deuxième angle conique (54).

5. Stent greffe prothétique selon la revendication 1, dans lequel le premier angle conique (32) est approximativement égal au deuxième angle conique (54).

6. Stent greffe prothétique selon la revendication 1, dans lequel le diamètre déployé de la deuxième admission (42) de la section de branche (40) est au moins aussi grand ou plus grand que le diamètre déployé du premier (20) ou deuxième (22) canal d'écoulement adjacent au point de bifurcation (15) de la section de tronc (12).

7. Stent greffe prothétique selon la revendication 1, dans lequel un diamètre déployé de la première (16) ou deuxième (18) évacuation est au moins le même ou plus petit que le diamètre déployé de la section de branche à une distance de la deuxième (42) admission égale à celle où se trouve la première (16) ou deuxième (18) évacuation du point de bifurcation (15).

8. Stent greffe prothétique selon la revendication 1, dans lequel la greffe vasculaire bifurquée de la section de tronc (12) entoure et est fixée aux premières une ou plusieurs sections de stent (24).

9. Stent greffe prothétique selon la revendication 1, dans lequel la greffe vasculaire tubulaire de la section de branche (40) entoure et est fixée aux deuxièmes une ou plusieurs sections de stent (50).
